# EUROPEAN PATENT APPLICATION

(11) **EP 1 803 402 A1**
(43) Date of publication of application: **04.07.2007**
(21) Application number: 05795865.4
(22) Date of filing: 20.10.2005
(51) Int. Cl.: A61B 8/00

(54) **ULTRASONIC DIAGNOSTIC EQUIPMENT AND CONTROL METHOD THEREFOR**

(30) Priority: 20.10.2004 JP 2004306125; 28.10.2004 JP 2004314345
(71) Applicant: KABUSHIKI KAISHA TOSHIBA, Tokyo 105-8001 (JP); Toshiba Medical Systems Corporation, Otawara-shi, Tochigi-ken 324-8550 (JP)
(72) Inventor: NAKAYA, Shigemitsu Toshiba Med.Systems Corporation, Tochigi 324-8550 (JP); KATAGUCHI, Muneki Toshiba Med.Systems Corporation, Tochigi 324-8550 (JP); ICHIOKA, Kenichi Toshiba Med.Systems Corporation, Tochigi 324-8550 (JP); KAKEE, Akihiro Toshiba Med.Systems Corporation, Tochigi 324-8550 (JP); TAKIMOTO, Masao Toshiba Med. Systems Corporation, Tochigi 324-8550 (JP); SUMI, Atsushi Toshiba Med. Systems Corporation, Tochigi 324-8550 (JP)
(74) Representative: Kramer - Barske - Schmidtchen
(86) International application number: PCT/JP2005/019321
(87) International publication number: WO 2006/043639

(57) **Abstract**

Three times of ultrasonic transmissions are executed in positive, negative and negative polarities with reference to a predetermined rate period, for each of a plurality of ultrasonic scan lines, and echo signals caused and generated by the ultrasonic waves of the respective transmissions are received at the same rates. At each scan line, the echo signal based on the positive-polarity ultrasonic wave of the first transmission and the echo signal based on the negative-polarity ultrasonic wave of the second transmission are added up, thereby to generate an ordinary mode image. Besides, at each scan line, the echo signal based on the positive-polarity ultrasonic wave of the first transmission and the echo signal based on the negative-polarity ultrasonic wave of the third transmission are added up, thereby to generate puncture mode image data. The generated ordinary mode image and puncture mode image are displayed in a predetermined aspect.

## Description

### Technical Field

The present invention relates to enhancements in the reliability and accuracy of, for example, puncture under ultrasonic guide, and more particularly to an ultrasonic diagnostic equipment which is used for the betterment of the visibility of a puncture needle, and a method of controlling the equipment.

### Background Art

An ultrasonic diagnostic equipment is a medical image equipment with which the tomographic image of a soft tissue in a living body is noninvasively obtained from the surface of the body by an ultrasonic pulse echo method. As compared with other medical image equipment, the ultrasonic diagnostic equipment has such merits as being small-sized and inexpensive, affording a high safety without exposure to X-rays etc., and being capable of blood flow imaging, and it is extensively utilized for the heart, the abdomen and the urinary organs and in obstetrics and gynecology, etc.

In an image diagnosis utilizing the ultrasonic diagnostic equipment, it is well known that an image of very high quality as exhibits a high resolution and involves few artifacts (artifact noise) can be generated by extracting and imaging harmonic components caused by the nonlinear waveform propagations of the tissue and the nonlinear vibrations of a contrast medium (higher harmonic components which appear at frequencies being integral times as high as a fundamental frequency). A filter method is typical as a method which derives nonlinear components such as the harmonic components caused by the nonlinear vibrations.

Besides, a method which extracts the harmonic components by removing fundamental wave components more effectively than the above filter method has been known as stated in, for example, Iwao Abiru and Tomoo Kamakura: "Nonlinear Propagation of Ultrasonic Pulses" (Technical Report of the Institute of Electronics, Information and Communication Engineers, US89-23, p53). This is such that two sorts of ultrasonic pulses whose phases are inverted to each other are alternately transmitted to an identical ultrasonic scan line, and that two sorts of reception signals which correspond to the transmission ultrasonic pulses are added up. The method is called the "phase inversion method", and it is a very meritorious technique which exerts a canceling action on the fundamental wave components, thereby to permit the removal of the fundamental wave components having entered harmonic bands as can never be removed by the filter method, and which conversely demonstrates an addition intensifying action for the harmonic components.

On the other hand, the phase inversion method works accurately only in a case where the tissue being a propagation medium is stationary, or in a case where propagation paths within the tissue are the same, to the last. Accordingly, when the method is applied to the actual living body in which the motions of organs represented by the heart exist, displacements occur at the individual parts between the reception signals of 2 rates under the influence of the motions, with the result that the fundamental wave remains unerased and gives rise to motion artifacts on an image.

Besides, when the interval of the transmissions of the ultrasonic pulses, namely, a PRF (Pulse Reputaion Frequency: pulse repetition frequency) lengthens, the displacements of the individual parts become larger, and the influence of the motion artifacts becomes intenser. The motion artifacts conceal a part desired to be watched, and simultaneously degrades the image drastically. In the phase inversion method, accordingly, it is common practice to make a contrivance which prevents the motion artifacts from appearing, for example, setting the PRF so as not to become unnecessarily long.

Meanwhile, in a radio frequency ablation (RFA) which is the local cure of hepatocellular carcinoma, or in a biopsy which examines a hepatocellular tissue, puncture under ultrasonic guide is extensively performed, and it is important to accurately puncture a part of interest such as tumor. It is therefore required that a place up to which a needle is inserted within the living body during the puncture can be definitely grasped on an ultrasonic image.

Nevertheless, in the puncture under the ultrasonic guide, the ultrasonic image of the needle is often buried in the image of the living body. Accordingly, there is the problem that the place up to which the needle is inserted is difficult to grasp.

### Disclosure of Invention

The present invention has been made in view of the above circumstances, and it has for its object to provide an ultrasonic diagnostic equipment which has a function capable of enhancing the visibility of a puncture needle in, for example, puncture under ultrasonic guide, and a method of controlling the ultrasonic diagnostic equipment.

In order to accomplish the object, the invention adopts means as stated below.

The first viewpoint of the invention consists in an ultrasonic diagnostic equipment wherein, in order to image an inner part of a patient, an ultrasonic transmission is executed at a first time interval into the patient, thereby to obtain a first image, comprising a transmission/reception unit which performs a plurality of ultrasonic transmissions into the patient at a second time interval longer than the first time interval, and which receives echo signals corresponding to ultrasonic waves of the respective transmissions, from the patient; an image generation unit in which the echo signals corresponding to the respective transmission ultrasonic waves are added or subtracted therebetween, thereby to generate second image data; and a display unit which displays a second image on the basis of the second image data.

The second viewpoint of the invention consists in an ultrasonic diagnostic equipment comprising an ultrasonic probe which includes ultrasonic transducers that perform transmissions/receptions of ultrasonic waves in a plurality of ultrasonic scan directions within a patient in response to applied drive signals, respectively; a transmission control unit which supplies the drive signals to the ultrasonic transducers so as to perform a plurality of times of ultrasonic transmissions which are taken with reference to a predetermined rate period and which include at least one time of polarity inversion, in each of the plurality of ultrasonic scan directions; a reception unit which receives a plurality of reflection waves based on the plurality of times of transmitted ultrasonic transmissions, with reference to the predetermined rate period; an image generation unit in which an addition or subtraction process is performed using two of the plurality of reflection waves as are temporally separate, at least, 2 rates at the predetermined rate period, thereby to generate first image data; and a display unit which displays a first image on the basis of the first image data.

The third viewpoint of the invention consists in a method of controlling an ultrasonic diagnostic equipment wherein, in order to image an inner part of a patient, an ultrasonic transmission is executed at a first time interval into the patient, thereby to obtain a first image, comprising performing a plurality of ultrasonic transmissions into the patient at a second time interval longer than the first time interval, and receiving echo signals corresponding to ultrasonic waves of the respective transmissions, from the patient; adding or subtracting the echo signals corresponding to the respective transmission ultrasonic waves, between them, thereby to generate second image data; and displaying a second image on the basis of the second image data.

The fourth viewpoint of the invention consists in an ultrasonic-diagnostic-equipment controlling method comprising supplying drive signals to ultrasonic transducers so as to perform a plurality of times of ultrasonic transmissions which are taken with reference to a predetermined rate period and which include at least one time of polarity inversion, in each of a plurality of ultrasonic scan directions within a patient; receiving a plurality of reflection waves which are based on the plurality of times of transmitted ultrasonic transmissions, with reference to the predetermined rate period; performing an addition or subtraction process by using those two of the plurality of reflection waves which are temporally separate at least 2 rates at the predetermined rate period, thereby to generate first image data; and displaying a first image on the basis of the first image data.

### Brief Description of Drawings

FIG. 1 is a diagram showing an example of the block configuration of an ultrasonic diagnostic equipment according to a first embodiment.
FIG. 2 is a conceptual diagram for explaining a scan sequence which is executed by a puncture mode function according to the first embodiment.
FIG. 3 is a conceptual diagram for explaining the signal processing of ultrasonic echo signals which are acquired by the puncture mode function according to the first embodiment.
FIG. 4 is a diagram showing an example of an ultrasonic image display which is executed by the puncture mode function.
FIG. 5 is a diagram showing another example of the ultrasonic image display which is executed by the puncture mode function.
FIG. 6 is a flow chart showing the flow of respective processing steps which are executed by the puncture mode function.
FIG. 7 is a conceptual diagram for explaining a scan sequence which is executed by a puncture mode function according to a second embodiment.
FIG. 8 is a conceptual diagram for explaining the signal processing of ultrasonic echo signals which are acquired by the puncture mode function according to the second embodiment.
FIG. 9 is a conceptual diagram for explaining a scan sequence which is executed by a puncture mode function according to a third embodiment.
FIG. 10 is a conceptual diagram for explaining the signal processing of ultrasonic echo signals as is executed by the puncture mode function according to the third embodiment.
FIG. 11 is a conceptual diagram for explaining a scan sequence which is executed by a puncture mode function according to a fourth embodiment.
FIG. 12 is a conceptual diagram for explaining the signal processing of ultrasonic echo signals as is executed by the puncture mode function according to the fourth embodiment.
FIG. 13 is a conceptual diagram for explaining a scan sequence which is executed by a manual puncture mode function according to a fifth embodiment.
FIG. 14 is a conceptual diagram for explaining the signal processing of ultrasonic echo signals which are acquired by the manual puncture mode function according to the fifth embodiment.
FIG. 15 is a flow chart showing the flow of respective processing steps which are executed in a manual puncture mode according to the fifth embodiment.
FIG. 16 is a block diagram showing the configuration of an ultrasonic diagnostic equipment according to a sixth embodiment.
FIG. 17 is a flow chart showing an operation in the ultrasonic diagnostic equipment according to the sixth embodiment.
FIG. 18A is a diagram showing an ultrasonic image which is displayed on a monitor 25 during a puncture mode in the sixth embodiment. Besides, FIG. 18B is a diagram showing an ultrasonic image which is displayed on the monitor 25 during a puncture mode in the prior art.
FIG. 19 is a diagram showing the images of a puncture needle as have been peak-held by the above processing at the procession and retrocession of the puncture needle within a patient.
FIG. 20 shows a diagram in the case where, when the puncture needle has deviated from the display angle of a puncture guideline, the angle of the puncture guideline is automatically corrected so as to be redisplayed.
FIG. 21 is a diagram showing a puncture guideline which is displayed on the monitor when the puncture needle has bent in the shape of a curve, in the sixth embodiment.

### Best Mode for Carrying Out the Invention

Now, the first embodiment through fourth embodiment of the present invention will be described in conjunction with the drawings. By the way, in the ensuing description, constituents which have substantially the same functions and constructions shall be assigned the same reference numerals and signs, and they shall be repeatedly described only on necessary occasions.

### (First Embodiment)

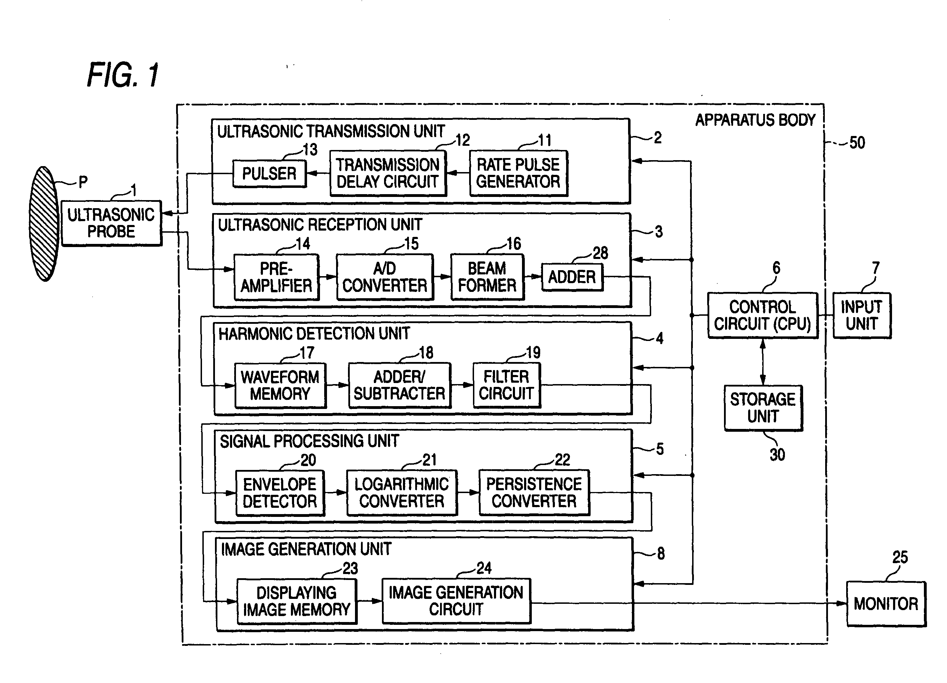

FIG. 1 shows a block configuration diagram of an ultrasonic diagnostic equipment according to this embodiment. As shown in the figure, the ultrasonic diagnostic equipment is configured of an ultrasonic probe 1, a storage unit 30, an input unit 7, a monitor 25 and an apparatus body 50.

The ultrasonic probe 1 generates ultrasonic waves and transmits them to a patient, and it receives reflection waves reflected from within the patient and generates echo signals. It has piezoelectric transducers which are acoustoelectric reversible transducers of piezoelectric ceramics or the like. The plurality of piezoelectric transducers are arrayed in parallel, and are disposed at the distal end of the ultrasonic probe 1.

The storage unit 30 stores therein images formed in the past, images accepted into the equipment from a network or a detachable type storage medium, dedicated programs for executing predetermined imaging sequences, and so forth.

The input unit 7 includes on its control panel, a liquid-crystal display panel and input devices such as a keyboard, a track ball, a mouse, and a dedicated I/F for executing a "puncture mode" to be explained later. An operator performs the inputting of transmission/reception conditions such as patient information and a rate period Tr, the selection of an image display mode, etc. by using the input unit 7.

The monitor 25 displays morphological information or blood flow information within the living body as an image on the basis of a video signal accepted from the apparatus body 50. The image, etc. displayed on the monitor 25 are stored in the storage unit within the apparatus body 50, in response to predetermined manipulations from the input unit 7, etc.

The apparatus body 50 includes an ultrasonic transmission unit 2 which performs the transmission control of the ultrasonic wave transmitted from the ultrasonic probe 1, an ultrasonic reception unit 3 which performs preprocessing for the echo signal received by the ultrasonic probe 1, a harmonic detection unit 4 which detects harmonic components from the preprocessed echo signal, a signal processing unit 5 which generates image data by subjecting the detected harmonic components to predetermined signal processing, an image generation unit 8 which generates the ultrasonic image on the basis of the image data so as to display this image, and a control circuit (CPU) 6.

The ultrasonic transmission unit 2 includes a rate pulse generator 11, a transmission delay circuit 12 and a pulser 13. The rate pulse generator 11 generates rate pulses which determine the repetition period (rate period) of ultrasonic pulses to be radiated into the patient, and it supplies the rate pulses to the transmission delay circuit 12. Subsequently, the transmission delay circuit 12, which is configured of independent delay circuits of M channels being equal in number to the ultrasonic transducers for use in the transmissions, endows the received rate pulses with focusing delay times for focusing the ultrasonic pulses on a predetermined depth, and deflecting delay times for transmitting the ultrasonic pulses into a predetermined direction, and it supplies the resulting rate pulses to the pulser 13. Besides, the pulse 13 has independent drive circuits of M channels being equal in number to the channels of the transmission delay circuit 12. A drive signal generated by each of the drive circuits is applied to the corresponding ultrasonic transducer disposed in the ultrasonic probe 1, whereby each of the ultrasonic transducers is driven so as to radiate the ultrasonic pulse into the patient.

The ultrasonic reception unit 3 includes a preamplifier 14, an A/D converter 15, a beam former 16 and an adder 28. The preamplifier 14 is designed so as to amplify a minute signal converted into an electrical reception signal by the ultrasonic transducer and to ensure a satisfactory S/N ratio. The fundamental wave component and harmonic components of the reception signal amplified to a predetermined magnitude in the preamplifier 14 are converted by the A/D converter 15 into digital signals, which are delivered to the beam former 16. The beam former 16 endows the reception signals converted into the digital signals, with focusing delay times for focusing the ultrasonic reflection waves from the predetermined depth, and deflecting delay times for scanning the patient by sequentially altering the reception directivities of the ultrasonic reflection waves. The adder 28 subjects the resulting outputs from the beam former 16, to a phasing addition (in which the reception signals obtained from the predetermined direction are added in inphase fashion).

The harmonic extraction unit 4 includes a waveform memory 17, an adder/subtracter 18 and a filter circuit 19. The waveform memory 17 once stores therein a reception signal which is obtained by first time of transmission/reception in a predetermined direction. The adder/subtracter 18 adds or subtracts reception signals obtained by second time - nth time (where n denotes a natural number of at least two, and n = 4 is assumed) of transmissions/receptions in the predetermined direction, and the reception signal saved in the waveform memory 17. On the other hand, the filter circuit 19 is a filter which reduces the fundamental component which could not be erased by a phase inversion method due to such causes as the motions of organs and body motions.

The signal processing unit 5 includes an envelope detector 20, a logarithmic converter 21 and a persistence converter 22. The envelope detector 20 subjects inputted digital signals to the calculation of envelope detection, thereby to detect an envelope. Besides, the logarithmic converter 21 includes a lookup table which performs the logarithmic conversion of an input value and outputs the resulting value, and the amplitudes of the reception signals are logarithmically converted in the logarithmic converter 21 so as to relatively emphasize weak signals. The persistence converter once stores scan lines of several frames in a memory, and it performs the processing of averaging brightness changes.

The image generation unit 8 includes a displaying image memory 23 and an image generation circuit 24. In the displaying image memory 23, image data supplied from the signal processing unit 5 and annexed data such as characters and numerals relevant to the image data are composited and are once saved. Besides, in the displaying image memory 23, image data as which an ordinary mode image and a puncture mode image to be explained later are composited in a predetermined aspect are once saved. The saved image data and annexed data are subjected to D/A conversion and television format conversion in the image generation circuit 24 so as to be displayed on the CRT monitor 25.

The control circuit 6 reads out the transmission/reception conditions and dedicated program stored in the storage unit 30, on the basis of the user's instructions of mode selection, transmission start/end, etc. inputted from the input unit 7, and it controls the individual units and the whole system statically or dynamically in accordance with the readout conditions, etc.

Especially in this embodiment, the control circuit 6 reads out the dedicated program for incarnating a puncture mode function to be explained later, from the storage unit 30, and expands the program on a predetermined memory, whereupon it executes the controls of the individual units in accordance with the program. By way of example, the control circuit 6 supplies the ultrasonic transmission unit 2 with a changeover control signal for the polarity of a drive pulse in the pulser 13, and it supplies the harmonic extraction unit 4 with control signals for determining filter characteristics, such as a center frequency and a frequency band, in the filter circuit 19, and addition/subtraction control signals in the waveform memory 17 and the adder/subtracter 18.

### (Puncture Mode Function)

Next, there will be described the puncture mode function which the ultrasonic diagnostic equipment has. This function images the motion of a puncture needle as a motion artifact by utilizing the phase inversion method. By the way, in this embodiment, an image condition under which a PRF is lengthened to deliberately generate the motion artifact, whereby the visibility of the puncture needle can be enhanced though this is unsuitable for the diagnosis of an in-vivo image, shall be called "puncture mode". Besides, an image condition which has heretofore been employed for ordinary diagnoses shall be called "ordinary mode".

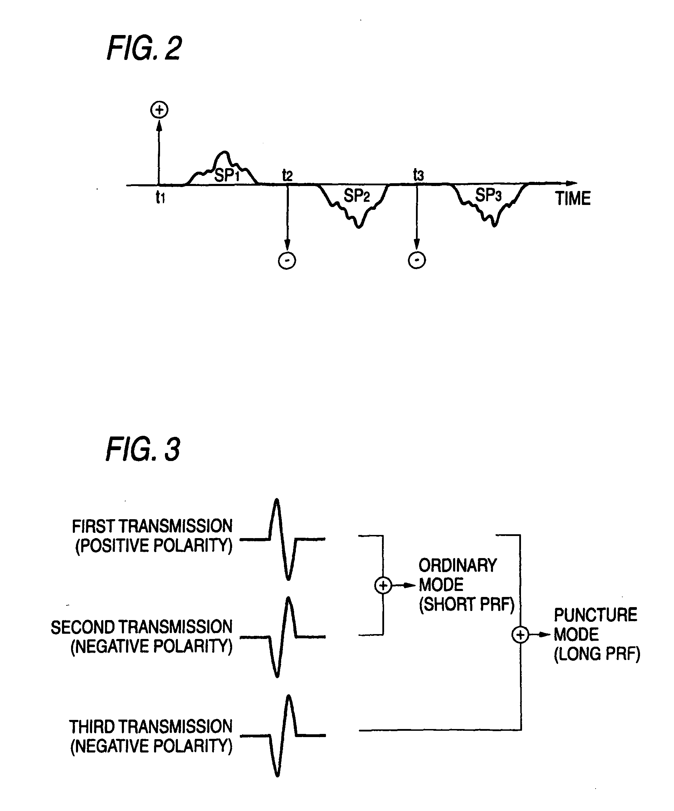

FIG. 2 is a conceptual diagram for explaining a scan sequence which is executed by the puncture mode function. As shown in the figure, in the imaging based on the puncture mode, three times of ultrasonic transmissions in which polarities are changed to be positive (time t₁), negative (time t₂) and negative (time t₃) for each of scan lines are executed at a predetermined PRF. Incidentally, SP₁, SP₂ and SP₃ in FIG. 2 simulatively indicate the spectral waveforms of echo signals which are obtained by the last ultrasonic transmissions.

FIG. 3 is a conceptual diagram for explaining the signal processing of the ultrasonic echo signals which are acquired by the puncture mode function. As shown in the figure, ordinary mode image data for generating an ultrasonic image (ordinary mode image) for use in a diagnosis are generated in, for example, the adder/subtracter 18 in such a way that the echo signal corresponding to the first time of ultrasonic transmission (that is, of the ultrasonic wave having the positive polarity at the time t₁ in FIG. 2), and the echo signal corresponding to the second time of ultrasonic transmission (that is, of the ultrasonic wave having the negative polarity at the time t₂ in FIG. 2) are added up for each of the scan lines.

In contrast, puncture mode image data for generating an ultrasonic image (puncture mode image) for grasping the motion of the puncture needle are generated in, for example, the adder/subtracter 18 in such a way that the echo signal corresponding to the first time of ultrasonic transmission, and the echo signal corresponding to the third time of ultrasonic transmission (that is, of the ultrasonic wave having the negative polarity at the time t₃ in FIG. 2) are added up.

Here, a difference to be stated below exists between the generation of the ordinary mode image data and that of the puncture mode image data. The ordinary mode image data are generated using the PRF which corresponds to an ordinary comparatively-short period. Therefore, the motion artifacts which are ascribable to the body motions, etc. between the first time of ultrasonic transmission and the second time of ultrasonic transmission occur at low possibilities, and ultrasonic image data capable of appropriately imaging organs, etc. can be generated. On the other hand, the puncture mode image data are generated using the PRF which corresponds to a length double the length of the ordinary mode image data. It is therefore possible to generate ultrasonic image data capable of appropriately imaging the motion artifacts which are ascribable to the body motions and the motion of the puncture needle occurring between the first time of ultrasonic transmission and the third time of ultrasonic transmission.

The ordinary mode image data are subjected to predetermined signal processing at the succeeding filter circuit 19 and signal processing unit 5, and are displayed on the monitor 5 as the ordinary mode image. Also, the puncture mode image data are subjected to similar processing and are displayed on the monitor 5 as the puncture mode image.

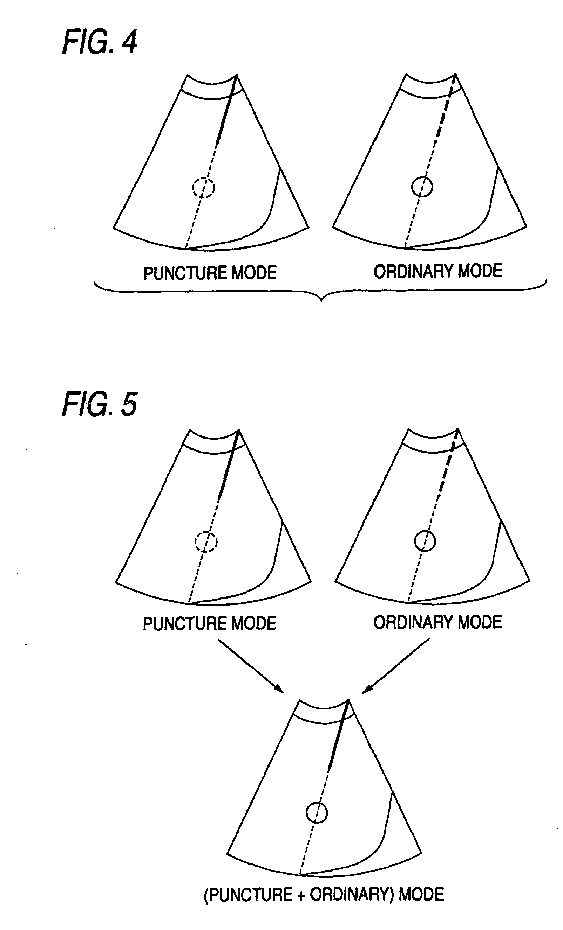

FIG. 4 is a diagram showing an example of an ultrasonic image display which is executed by the puncture mode function. As shown in the figure, on the monitor 5, the ordinary mode image and the puncture mode image are simultaneously displayed in parallel by way of example. While confirming the position of the puncture needle on the puncture mode image, an operator can observe the state of the organ on the ordinary mode image and can treat the organ. Especially during an operation, a brightness change on the ultrasonic image is sometimes generated by moving the puncture needle slightly, thereby to enhance the visibility of the puncture needle. Since the puncture mode image has been created using the two signals which are temporally more separate than usual, the motion artifact is represented more remarkably than in the prior art. Accordingly, the operator can visually recognize the position of the puncture needle on the ultrasonic image more definitely than in the prior art.

FIG. 5 is a diagram showing another example of the ultrasonic image display which is executed by the puncture mode function. As shown in the figure, on the monitor 5, the ordinary mode image and the puncture mode image are displayed in superposition by way of example, whereby the same object can be attained.

By the way, in the puncture mode, the image processing items of a dynamic range, a gain, postprocessing, persistence, etc. should preferably be made different from those of the ordinary mode in order to enhance the visibility of the puncture needle.

Besides, in order to keep a visibility even when the motion of the needle has stopped, the equipment may well be configured so as to separately perform a peak holding process (maximum-brightness-value holding process) which uses images of several frames.

Further, in order to facilitate recognizing the position of the puncture needle, the equipment may well be configured so as to display at least part of the puncture mode image (for example, a region containing the puncture needle) in a color which differs from the color of the surroundings of the part or the ordinary mode image.

### (Operation)

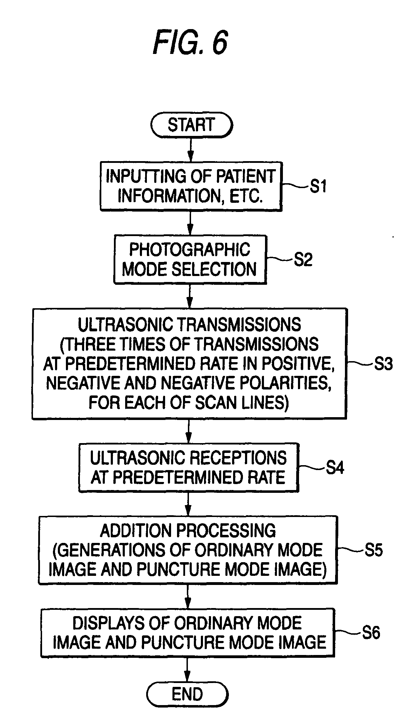

Next, there will be described an operation in the puncture mode of the ultrasonic diagnostic equipment. FIG. 6 is a flow chart showing the flow of processing steps which are executed in the puncture mode. As shown in the figure, patient information, a diagnostic part, etc. are first inputted (step S1), and the selection of an imaging mode (here, the selection of the "puncture mode") is made (step S2).

Subsequently, three times of ultrasonic transmissions are executed in positive, negative and negative polarities with reference to a predetermined rate period, for each of a plurality of ultrasonic scan lines (scan directions) (step S3), and reflected waves caused and generated by respective ultrasonic transmission waves are received at the same rate (step S4).

Subsequently, at each scan line, an echo signal based on the ultrasonic wave of the positive polarity at the first time of ultrasonic transmission and an echo signal based on the ultrasonic wave of the negative polarity at the second time of ultrasonic transmission are added up, thereby to generate ordinary mode image data. Further, at each scan line, the echo signal based on the ultrasonic wave of the positive polarity at the first time of ultrasonic transmission and an echo signal based on the ultrasonic wave of the negative polarity at the third time of ultrasonic transmission are added up, thereby to generate puncture mode image data (step S5).

Subsequently, an ordinary mode image and a puncture mode image are displayed in a predetermined aspect on the basis of the respective generated image data (step S6).

According to the configuration described above, advantages to be stated below can be obtained.

According to the ultrasonic diagnostic equipment, in the imaging which utilizes the phase inversion method, the second image for deliberately imaging a motion artifact is generated using at least two reflected waves which are temporally more separate than at least two reflected waves used for the diagnostic image (first image). In, for example, a treatment employing the puncture needle, the position of the puncture needle can be grasped more definitely than in the prior art by utilizing the second image, that is, by observing the first image and the second image which are displayed in parallel or in superposition. As a result, the ultrasonic diagnostic equipment can contribute to the offer of medical care of high quality.

Moreover, according to the ultrasonic diagnostic equipment, any operation other than selecting the puncture mode first is not required for imaging the puncture needle. Accordingly, the operator can observe the ultrasonic image in which the puncture needle is imaged more definitely, without performing any special operation, and an operating burden on him/her during the operation can be relieved.

Besides, the puncture mode function which is incarnated by the ultrasonic diagnostic equipment can be realized in such a way that the dedicated program for incarnating the function is installed in an existing ultrasonic system. Accordingly, a safe puncture needle, etc. can be realized easily and at a low cost.

### (Second Embodiment)

Next, the second embodiment of the invention will be described. This embodiment consists in that imaging which enhances the visibilities of a puncture needle, etc. is performed by further utilizing a rate subtraction method. Incidentally, the "rate subtraction method" is a technique which performs the imaging by utilizing the difference between inphase images (images in the same polarity), and which is stated in, for example, JP-A-8-336527.

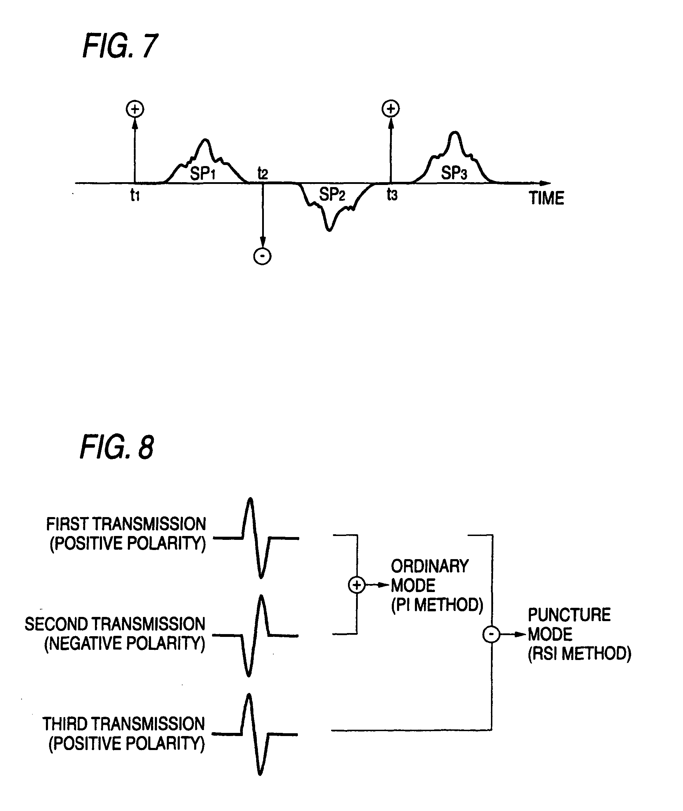

FIG. 7 is a conceptual diagram for explaining a scan sequence which is executed by a puncture mode function according to the second embodiment. Besides, FIG. 8 is a conceptual diagram for explaining the signal processing of ultrasonic echo signals as is executed by the puncture mode function according to the second embodiment.

As shown in FIG. 7, ultrasonic pulses of inverted polarities or positive, negative and positive polarities are transmitted three times, for each of a plurality of ultrasonic scan lines by way of example. By the way, in the figure, the echo signals obtained by the ultrasonic transmissions are respectively indicated by spectral waveforms as SP₁, SP₂ and SP₃.

In this embodiment, an ordinary mode image is generated on the basis of image data obtained by the addition between the echo signal SP₁ (positive polarity) and the echo signal SP₂ (negative polarity), as in the first embodiment. On the other hand, a puncture mode image is generated on the basis of image data obtained by the subtraction between the echo signal SP₁ (positive polarity) and the echo signal SP₃ (positive polarity). On this occasion, in the puncture mode image, parts which do not move between the first ultrasonic transmission and the third ultrasonic transmission are not imaged by the subtraction processing. However, in a case where the puncture needle is moving between them, a remaining motion artifact component is appropriately imaged by the difference.

With such a configuration, the same advantages as in the first embodiment can be attained, and the object can be accomplished.

### (Third Embodiment)

Next, the third embodiment of the invention will be described. This embodiment consists in that imaging which enhances the visibilities of a puncture needle, etc. is performed by utilizing a dummy rate. Incidentally, the "dummy rate" is a rate at which an ultrasonic transmission is performed, but imaging utilizing a reflection wave obtained by the ultrasonic transmission is not performed, or at which the ultrasonic transmission itself is not performed.

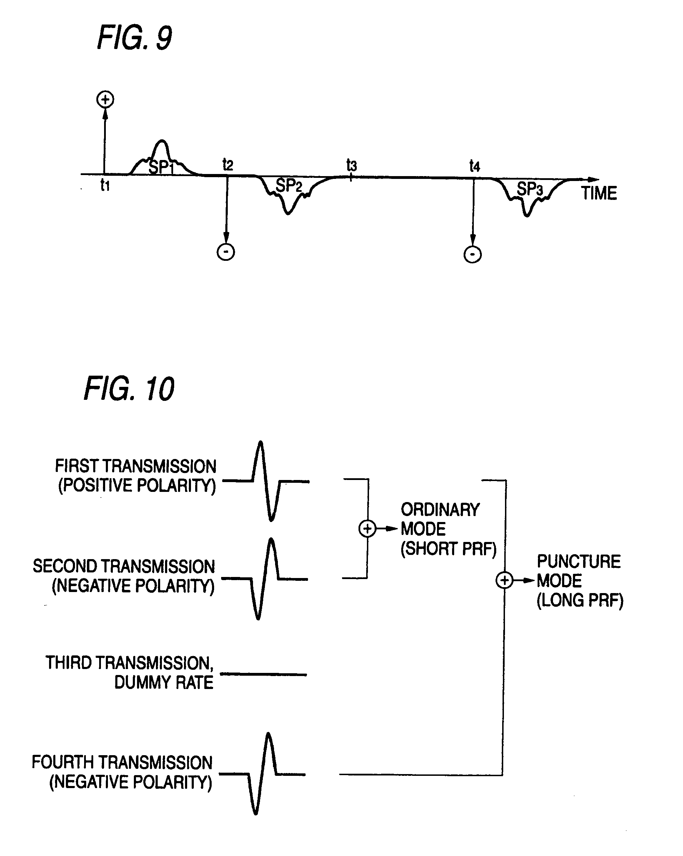

FIG. 9 is a conceptual diagram for explaining a scan sequence which is executed by a puncture mode function according to the third embodiment. Besides, FIG. 10 is a conceptual diagram for explaining the signal processing of ultrasonic echo signals as is executed by the puncture mode function according to the third embodiment.

In this embodiment, as shown in FIG. 9 by way of example, three times of ultrasonic transmissions in positive polarity, in negative polarity, at the dummy rate and in negative polarity are performed for each of a plurality of ultrasonic scan lines. Besides, in this embodiment, as shown in FIG. 10, an ordinary mode image is generated on the basis of image data obtained by the addition between a reflection wave SP₁ (positive polarity) and a reflection wave SP₂ (negative polarity), as in the first or second embodiment. On the other hand, a puncture mode image is generated on the basis of image data obtained by the addition between the reflection wave SP₁ (positive polarity) of the first transmission and the reflection wave SP₃ (negative polarity) of the third transmission preceded by the dummy rate.

Owing to such intervention of the dummy rate, a PRF can be lengthened one time of ultrasonic pulse transmission more than in the case of the first or second embodiment. Accordingly, a larger number of motion artifacts appearing meantime can be imaged, and the visibility of the puncture needle can be enhanced.

Incidentally, this embodiment may well be configured so as to generate a puncture mode image in such a way that the rate subtraction method is performed by setting the same polarity for the ultrasonic waves of the first and third transmissions.

### (Fourth Embodiment)

Next, the fourth embodiment of the invention will be described. This embodiment is another example which utilizes a dummy rate.

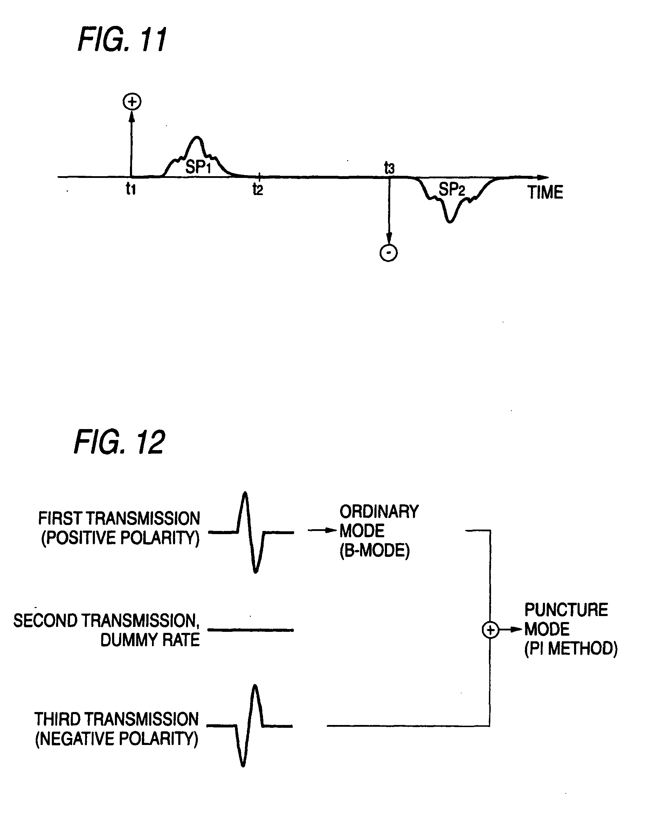

FIG. 11 is a conceptual diagram for explaining a scan sequence which is executed by a puncture mode function according to the fourth embodiment. Besides, FIG. 12 is a conceptual diagram for explaining the signal processing of ultrasonic echo signals as is executed by the puncture mode function according to the fourth embodiment.

In this embodiment, as shown in FIG. 11 by way of example, two times of ultrasonic transmissions in positive polarity, at the dummy rate and in negative polarity are performed for each of a plurality of ultrasonic scan lines. An ordinary mode image is formed by imaging in a B-mode without phase inversion, in only the positive polarity of the first transmission, while a puncture mode image is formed by imaging employing the positive-polarity ultrasonic wave of the first transmission and the negative-polarity ultrasonic wave of the third transmission preceded by the dummy rate.

Owing to the intervention of the dummy rate as in the third embodiment, a PRF can be lengthened one time of ultrasonic pulse transmission more. Accordingly, a larger number of motion artifacts appearing meantime can be imaged, and the visibility of the puncture needle can be enhanced.

Incidentally, this embodiment may well be configured so as to generate a puncture mode image in such a way that the rate subtraction method is performed by setting the same polarity for the ultrasonic waves of the first and second transmissions.

By the way, the invention is not restricted to the above embodiments as they are, but it can be embodied by modifying constituents within a scope not departing from the purport thereof, at the stage of performance. A practicable modification is, for example, as stated below.

### (Fifth Embodiment)

Next, the fifth embodiment of the invention will be described. Each of the first through fourth embodiments has adopted the technique which lengthens the PRF and deliberately generates the motion artifacts in order to enhance the visibility of the puncture needle, though such is unsuitable for the diagnosis of an in-vivo image. In contrast, this embodiment elucidates an imaging technique according to which, in the phase inversion method, an ultrasonic transmission for imaging the puncture needle is performed upon lapse of a desired delay time T after a reference ultrasonic transmission, for each scan line, whereupon the motion artifacts are deliberately generated by employing the needle imaging ultrasonic transmission.

In this embodiment, an imaging mode in which the motion artifacts are deliberately generated by setting the delay time T, whereby the visibility of the puncture needle can be enhanced though such is unsuitable for the diagnosis of the in-vivo image, shall be called "manual puncture mode" below.

The block configuration of an ultrasonic diagnostic equipment 1 according to this embodiment is substantially the same as shown in FIG. 2. Now, there will be described only constituents whose functions are different from the functions in the embodiments already described.

A storage unit 30 stores therein a dedicated program for incarnating a manual puncture-needle imaging mode to be described later, and parameters for use in the manual puncture-needle imaging mode.

A control circuit 6 reads out the dedicated program stored in the storage unit 30, and it expands this program on a memory not shown, so as to incarnate the manual puncture mode function. The contents of the function will be described in detail later.

An ultrasonic transmission/reception unit 2 executes ultrasonic transmissions/receptions in accordance with set transmission conditions in the manual puncture mode.

An input unit 7 is an interface for setting the delay time T to be explained later, at a desired value.

### (Manual Puncture Mode Function)

Next, the manual puncture mode function will be described. In this mode, a reference ultrasonic transmission is set. It is selected as the start time of a delay time T. By way of example, in a case where n times of ultrasonic transmissions (here, n denotes an integer of at least 3) including at least one time of phase inversion are performed at each scan line, the first ultrasonic transmission or the ultrasonic transmission immediately before the ultrasonic transmission for imaging a puncture needle can be adopted as the reference ultrasonic transmission.

By the way, in this embodiment, in order to concretize the description, there will be supposed an example in which the manual puncture mode is applied to the scan sequence according to the first embodiment, that is, in which in the case of executing the three times of ultrasonic transmissions (the ultrasonic transmissions in the positive, negative and negative polarities) that include at least one time of phase inversion for each scan line, the puncture needle imaging ultrasonic transmission is executed at the third time in accordance with the delay time T that is set with reference to the first ultrasonic transmission. The manual puncture mode, however, may well be applied to, for example, the scan sequence according to any of the second through fourth embodiments, without sticking to the supposed example.

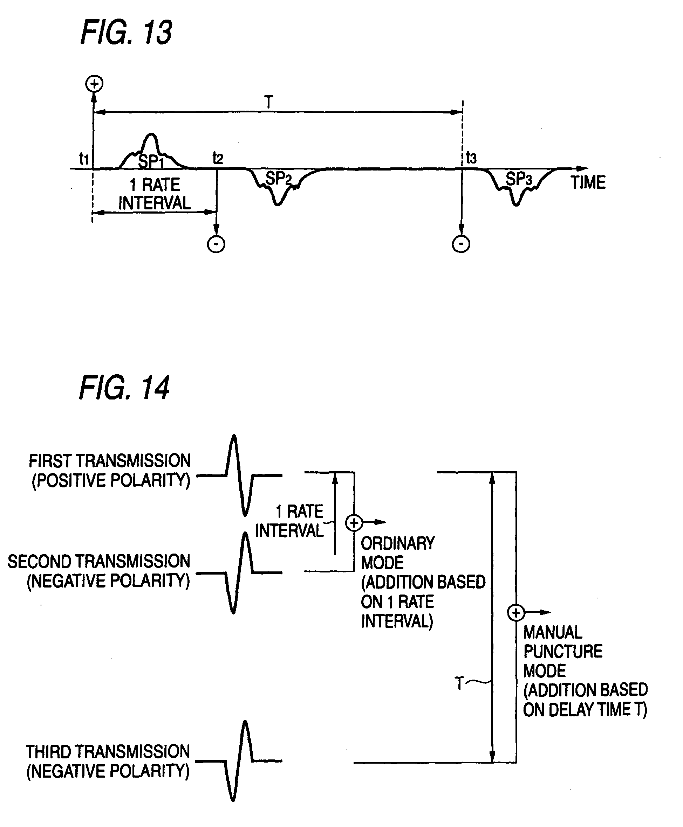

FIG. 13 is a conceptual diagram for explaining a scan sequence which is executed by the manual puncture mode function. As shown in the figure, in the imaging based on the manual puncture mode, three times of ultrasonic transmissions whose polarities are inverted to be positive (time t₁), negative (time t₂) and negative (time t₃) are executed for each of a plurality of scan lines. Here, the time interval between the time t₁ and the time t₂ is one rate interval, and the time interval between the time t₁ and the time t₃ is the delay time T. Accordingly, an operator sets the delay time T at a desired value by a predetermined manipulation, whereby he/she can execute the puncture needle imaging ultrasonic transmission at the time interval independent of the PRF interval, and he/she can deliberately generate motion artifacts by the imaging ultrasonic transmission so as to image the puncture needle.

FIG. 14 is a conceptual diagram for explaining the signal processing of ultrasonic echo signals which are acquired by the manual puncture mode function. As shown in the figure, ordinary mode image data for generating an ultrasonic image for use in a diagnosis (an ordinary mode image) are generated in, for example, the adder/subtracter 18 in such a way that the echo signal corresponding to the first transmission of an ultrasonic wave (that is, an ultrasonic wave having the positive polarity at the time t₁ in FIG. 13), and the echo signal corresponding to the second transmission of an ultrasonic wave (that is, an ultrasonic wave having the negative polarity at the time t₂ in FIG. 13) are added up for each scan line.

In contrast, image data for generating an ultrasonic image for grasping the motion of the puncture needle (a manual puncture mode image) are generated in, for example, the adder/subtracter 18 in such a way that the echo signal corresponding to the first transmission ultrasonic wave transmitted at the time t₁, and the echo signal corresponding to the third transmission ultrasonic wave transmitted upon lapse of the delay time T after the time t₁ (that is, the ultrasonic wave having the negative polarity at the time t₃ in FIG. 13) are added up. The filter circuit 19 and signal processing unit 5 at the succeeding stages subject the generated manual puncture mode image data to predetermined signal processing, thereby to generate the manual puncture mode image. The generated manual puncture mode image is displayed on the monitor 5 in, for example, the aspect shown in FIG. 4.

Here, a difference to be stated below exists between the generation of the puncture mode image data acquired in, for example, the first embodiment and the generation of the manual puncture mode image data acquired in this embodiment. The puncture mode image data are generated using the echo signals which correspond to the two transmission ultrasonic waves that have the time interval being integral times (in the first embodiment, double) the PRF. On the other hand, the manual puncture mode image data are generated using the echo signal which corresponds to the first ultrasonic wave transmitted at the time t₁, and the echo signal which corresponds to the third ultrasonic wave transmitted at the time t₃ upon the lapse of the delay time T after the time t₁. According to the manual puncture mode image data, the motion artifacts which are ascribable to the motion that occurs during the desired delay time T independent of the integral times of the PRF can be appropriately imaged.

Incidentally, also in the manual puncture mode, as in the case of the puncture mode, image processing relevant to a dynamic range, a gain, postprocessing, persistence, etc., a peak holding process, the color display of at least part of the image, and so forth should preferably be performed in order to enhance the visibility of the puncture needle.

### (Operation)

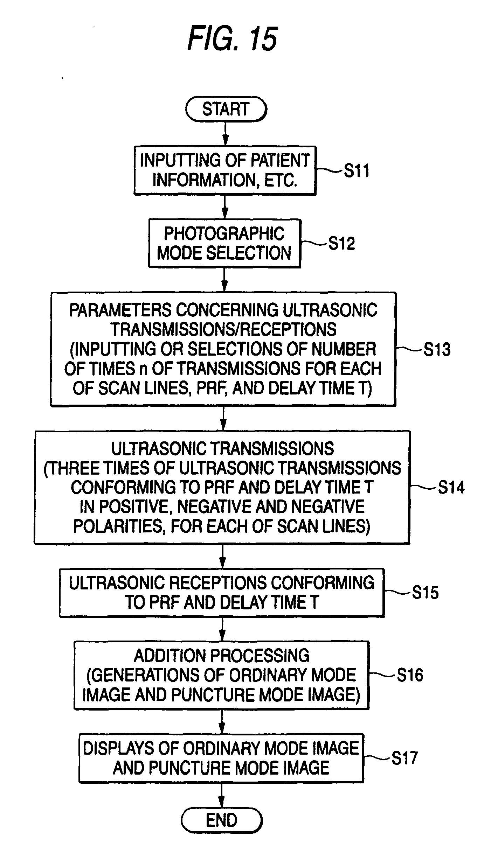

Next, there will be described an operation in the manual puncture mode of the ultrasonic diagnostic equipment. FIG. 15 is a flow chart showing the flow of processing steps which are executed in the manual puncture mode. As shown in the figure, patient information, a diagnostic part, etc. are first inputted (step S11), and the selection of an imaging mode (here, the selection of the "manual puncture mode") is made (step S12).

Subsequently, parameters concerning ultrasonic transmissions/receptions in the manual puncture mode are inputted (step S13). Here, the expression "parameters concerning ultrasonic transmissions/receptions in the manual puncture mode" signifies parameters which contain, at least, the number of times n of transmissions for each of scan lines, a PRF and/or a delay time T. Regarding the selections of the parameters, by way of example, the table of preset combinations each consisting of a plurality of set values is stored in the storage unit 30 beforehand, so as to select a desired one of the combinations from within the table.

Subsequently, three times of ultrasonic transmissions are executed in positive, negative and negative polarities in conformity with the PRF and delay time T as predetermined, for each of the plurality of ultrasonic scan lines (scan directions) (step S14), and reflection waves which are caused and generated by the respective transmission ultrasonic waves are received (step S15).

Subsequently, an echo signal based on the first transmission ultrasonic wave of the positive polarity and an echo signal based on the second transmission ultrasonic wave of the negative polarity are added up at each scan line, thereby to generate ordinary mode image data. Further, the echo signal based on the first transmission ultrasonic wave of the positive polarity and an echo signal based on the third transmission ultrasonic wave of the negative polarity are added up at each scan line, thereby to generate manual puncture mode image data (step S16). An ordinary mode image and a manual puncture mode image are respectively displayed in a predetermined aspect on the basis of the generated image data (step S17).

According to the configuration described above, advantages to be stated below are attained.

According to the ultrasonic diagnostic equipment, in a case where n times of ultrasonic transmissions (n being an integer of at least 3) which include at least one time of phase inversion are performed for each scan line, the puncture needle imaging ultrasonic transmission is executed upon lapse of the delay time T after the reference ultrasonic transmission. Accordingly, the ultrasonic transmission for imaging a puncture needle can be executed at a timing independent of the integral times of the PRF (that is, upon the lapse of the delay time T after the reference ultrasonic transmission), and motion artifacts ascribable to that motion of the puncture needle which occurs during the delay time T can be appropriately imaged.

Moreover, the delay time T can be set at the desired value of an operator by a predetermined manipulation. Accordingly, the operator can execute the puncture needle imaging ultrasonic transmission conforming to the desired delay time T, through the predetermined manipulation, and he/she can deliberately generate the motion artifacts by employing the imaging ultrasonic transmission, so as to image the puncture needle.

Besides, the manual puncture mode function which is incarnated by the ultrasonic diagnostic equipment can be realized in such a way that the dedicated program for incarnating the function is installed in an existing ultrasonic system. Accordingly, a safe puncture needle, etc. can be realized easily and at a low cost.

### (Sixth Embodiment)

Next, the sixth embodiment of the invention will be described. This embodiment enhances the visibility of a puncture needle on an image.

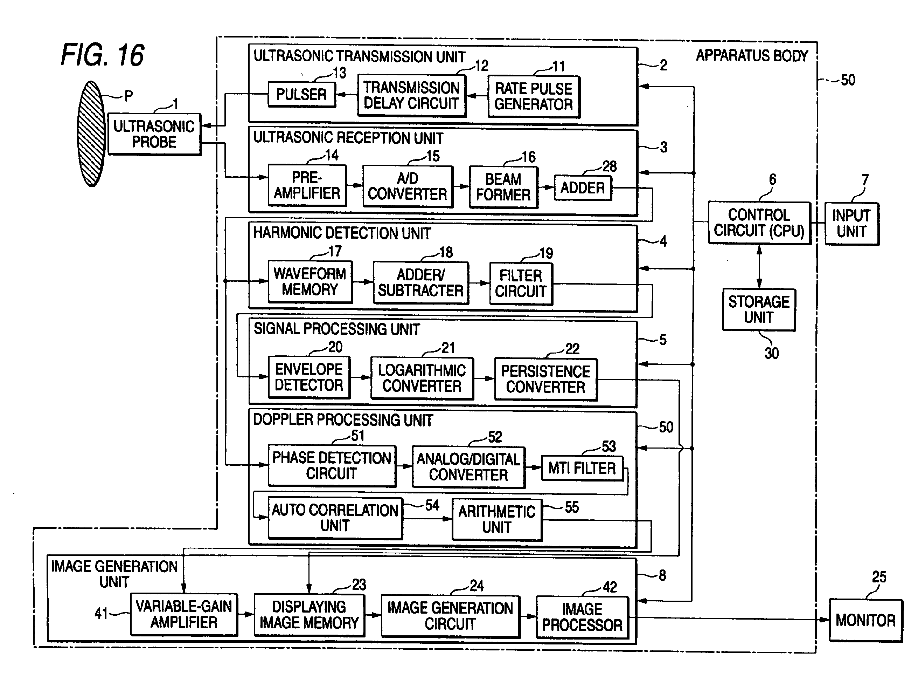

FIG. 16 is a block diagram showing the configuration of an ultrasonic diagnostic equipment according to this embodiment. As shown in the figure, the ultrasonic diagnostic equipment 1 according to this embodiment differs from the configuration shown in FIG. 1, in the point of further including an image processing unit 40 and a Doppler processing unit 50. Now, there will be described the image processing unit 40, and other constituents having functions different from the contents already stated.

The image processing unit 40 includes a variable-gain amplifier 41 and an image processor 42. The variable-gain amplifier 41 is for increasing or decreasing the gain of a Doppler image signal, and it is constructed of, for example, a multiplication type D/A converter. The image processor 42 is an image generation assistance unit which has the function of performing a peak holding process for a signal recognized as indicating the puncture needle, by using a B-mode image signal transmitted from a signal processing unit 5 and the Doppler image signal transmitted from the Doppler processing unit 50, and then displaying a locus, and the function of predicting the course of the puncture needle from the obtained locus, and automatically correcting a guide when the course has deviated from a puncture guideline. Here, the image processor 42 changes the gain of the variable-gain amplifier 41 so as to lower the color sensitivity of a color flow image.

In a diagnostic sequence to be explained later, a transmission circuit 13 transmits ultrasonic waves in accordance with voltage pulses, transmission frequencies, etc. conforming to various programmed transmission conditions.

An image generation circuit 24 divides a converted tomographic layer composed of the information items of respective scan lines, into pixels, and it assigns the respective pixels to memory addresses so as to store the reflection intensity information of the tomographic layer therein. Besides, in order to depict the puncture guideline indicative of the inserted position of the puncture needle, the image generation circuit 24 stores the dot positions of the puncture guideline as data therein.

The Doppler processing unit 50 is configured of a phase detection circuit 51, an analog/digital converter 52, an MTI filter 53, an autocorrelation unit 54 and an arithmetic unit 55, and it extracts blood flow components and the movement components of the tip of the puncture needle as are based on the Doppler effect, so as to obtain a mean velocity, a variance, power and the like Doppler image information items for multiple points. The Doppler image information items are sent to a monitor 25 through the image generation unit 8, and are color-displayed as a mean velocity image, a variance image and a power image or the combined image of these images.

The monitor 25 displays the tip of the puncture needle, the puncture guideline, etc. added to and composited with a tomographic image, in an aspect to be explained later.

### (Operation)

Next, the operation of the ultrasonic diagnostic equipment according to the invention will be described below with reference to the drawings.

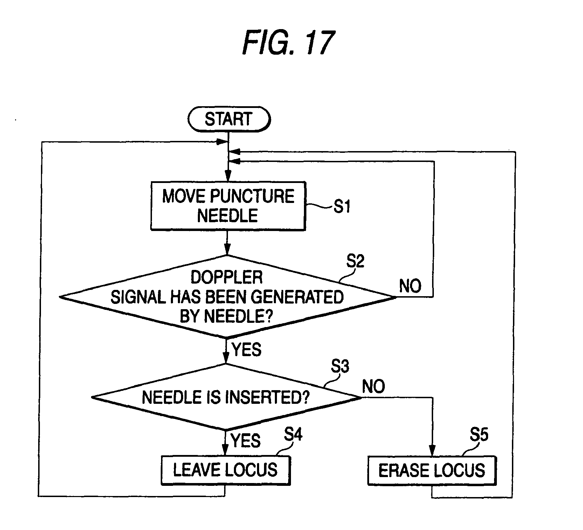

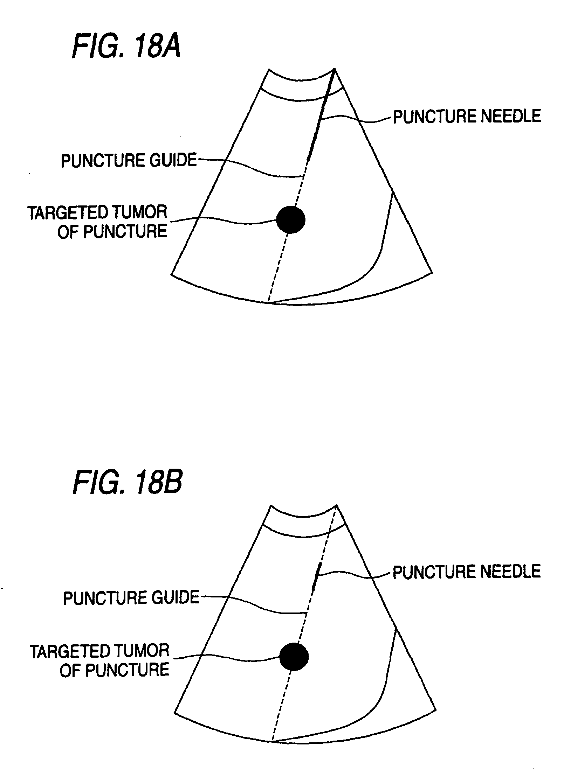

FIG. 17 is a flow chart showing the operation in an embodiment of the ultrasonic diagnostic equipment according to the invention. Besides, FIG. 18A is a diagram showing an ultrasonic image which is displayed on the monitor 25 during the execution of the puncture in this embodiment. Further, for reference, an ultrasonic image in the prior art as is displayed on the monitor 25 during the execution of puncture is shown in FIG. 18B.

As shown in FIG. 17, when the puncture needle has been first moved (S1), a Doppler signal is transmitted from the signal processing unit 5 and Doppler processing unit 50 to the image generation unit 8. The Doppler processing unit 50 decides whether or not the pertinent signal is a signal generated by the movement of the puncture needle (S2).

As shown in FIG. 18B, in the prior art, the image of the puncture needle is buried in the image of a living body, and it is difficult to grasp where the puncture needle lies. As shown in FIG. 18A, therefore, the puncture needle is recognized in such a way that the threshold decision is rendered in the image processor 42 by using the information items of a brightness and a position in a B-mode, the information items of a velocity, power and a traveling direction in a Doppler mode, etc. in the signal which has been transmitted from the signal processing unit 5 and Doppler processing unit 50 to the image generation unit 8.

Regarding a threshold value which the image processor 42 uses for the decision in order to recognize the puncture needle, the following conditions are mentioned by way of example:
(1) The brightness of the B-mode is high
(2) The signal is one near the puncture guideline
(3) A velocity component is contained in the Doppler mode
(4) The direction in the Doppler mode and a direction indicated by the puncture guideline are substantially in agreement
(5) A power value is large
A signal which satisfies at least any one of the conditions, is recognized as the puncture needle by the image processor 42. Besides, the threshold value is stored in the storage unit 30, and the image processor 42 reads out the threshold value from the storage unit 30 in the case of the threshold decision.

In general, the puncture needle exhibits a comparatively high brightness at only the needle tip (distal end part) and low brightnesses at the other parts, so that the image of the puncture needle is buried in the image of the living body. Accordingly, in the case where the Doppler signal satisfying the above condition has been recognized (S2-Yes), it is recognized as having been generated by the movement of the puncture needle, and the image processor 42 subjects the B-mode image signal of the tip of the puncture needle to the peak holding process. Thus, the locus of the tip of the puncture needle is regarded as the image of the whole puncture needle. Therefore, the image of the puncture needle becomes more definite, and even when the motion of the puncture needle has stopped to generate no Doppler component, it is possible to know the position at which the tip of the puncture needle lies, and a state in which the whole puncture needle is. Incidentally, the peak holding process by the image processor 42 is executed in any state of the puncture with the puncture needle (in any of all states where the puncture needle is inserted, stopped and pulled out). Besides, while the puncture needle is moving, a Doppler measurement in the prior art is not performed.

Here, in this embodiment, the recognized puncture needle may well be subjected to a coloring process by the image processor 42, thereby to enhance its visibility.

Besides, when the image processor 42 executes a process in which only the tip part of the puncture needle as recognized by the B-mode image signal is displayed in a different color, only the puncture needle tip can be emphasized, and the position to which the puncture needle has proceeded becomes more definite.

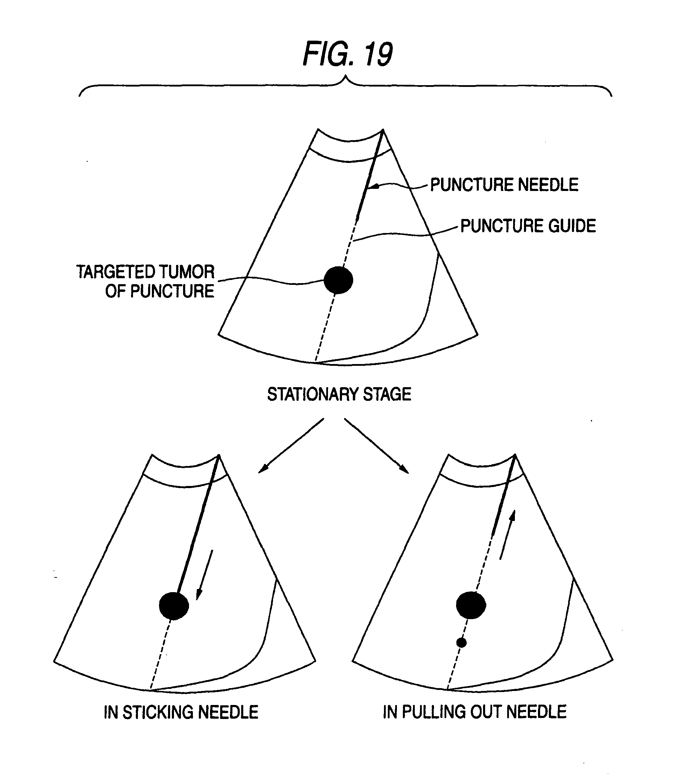

FIG. 19 is a diagram showing those images of a puncture needle which have been peak-held by the above processing in the procession and retrocession of the puncture needle within a patient.

In the puncture, there are performed the series of operations of sticking the puncture needle into a part to-be-punctured ("targeted tumor of puncture" in the figure) and thereafter pulling out the puncture needle. As indicated in the above condition (3), when the puncture needle is stuck, the Doppler image signal has the component which comes away from the surface of the body of the patient, and when the puncture needle is pulled out, the Doppler image signal has the component which comes near to the body surface.

Therefore, the image processor 42 executes processing in which, when the puncture needle is stuck, that is, when the Doppler image signal has the component coming away from the body surface (S3-Yes in FIG. 17), the locus of the puncture needle (tip) is left behind as stated before (S4 in FIG. 17), and in which, when the puncture needle is pulled out (S3-No in FIG. 17), that is, when it has been detected that the Doppler image signal has the component coming near to the body surface of the patient, a movement displacement component is erased relative to the locus of the tip of the puncture needle as subjected to the peak-holding display by the processing stated before (S5 in FIG. 17).

Thus, the locus of the puncture needle is prevented from remaining even after the puncture needle has been pulled out.

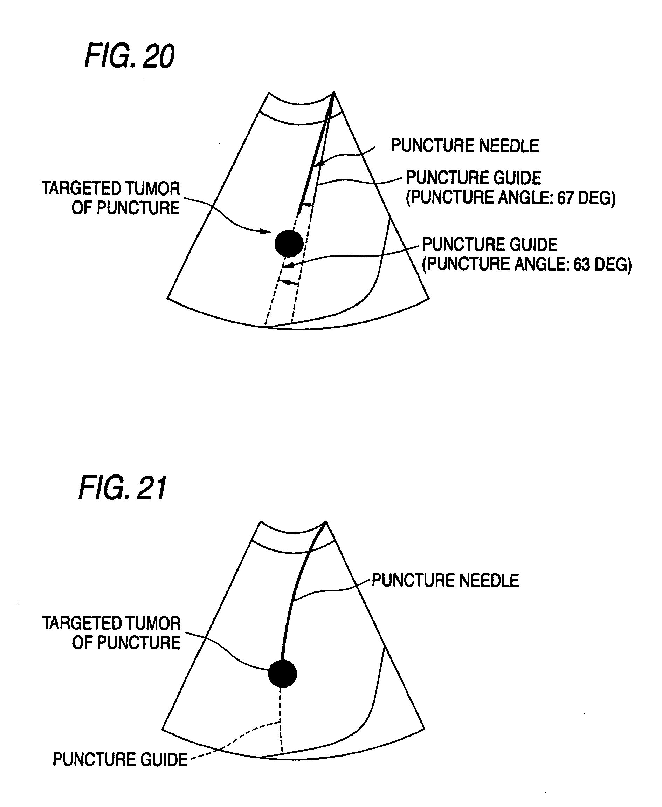

FIG. 20 shows a diagram in the case where, when a puncture needle has deviated from the display angle of a puncture guideline, the angle of the puncture guideline is automatically corrected so as to be redisplayed.

Usually, in a puncture mode, a line (for example, dotted line) called the "puncture guideline" is subjected to image generation processing by the image processing unit 40, and it is displayed in superposition on the monitor 25 (on an ultrasonic image) by the control circuit 6, whereby the line display can be compared with the display of the behavior of the puncture needle (the insertion or pulling-out of the puncture needle into or from a patient).

In some cases, however, the puncture needle undergoes a distortion under the influence of a tissue within the living body, and the puncture needle does not proceed in the same direction as a direction indicated by the puncture guideline.

Therefore, the processing of a linear regression calculation or the like is executed by the image processor 42 on the basis of the locus of the tip of the puncture needle as displayed by the foregoing processing, and the control circuit 6 predicts the proceeding direction of the puncture needle on the basis of a result obtained by the image processor 42 and corrects the deviation of the puncture guideline from the predicted direction so as to redisplay the puncture guideline on the monitor 25.

Concretely, the puncture guideline is displayed in such a way that the control circuit 6 executes a linear regression calculation on the basis of the positions of the puncture needle with the lapse of time, on the ultrasonic image as specified by the B-mode image signal, thereby to find a position at which the puncture guideline is to be displayed, whereupon the control circuit 6 writes the puncture guideline into the displaying image memory 23. That is, the positions (dots) of the tip of the puncture needle with the lapse of time, as specified by the B-mode image signal are considered as statistical data, and the linear regression calculation being one of statistic processes is executed, whereupon a straight line along which the puncture needle can be predicted to proceed in approximation to a straight line indicating the relation of the dots is displayed as the puncture guideline.

In this way, it is possible to curtail man-hour in the case where the correction processing of the ultrasonic image of the puncture needle and the position and angle of the puncture guideline as indicate the insertion position of the puncture needle is manually performed before the puncture into the patient. Moreover, since the puncture guideline can be corrected merely by disposing a linear regression calculation circuit, the correction can be made by an inexpensive unit.

Besides, the puncture guideline in this embodiment may well be displayed as two straight lines having an arbitrary angle therebetween, not as one straight line. In a case where the position and angle of the puncture guideline corrected here exceed those of the maximum guideline expectable as corrective values, the equipment may well prompt the operator to make a correction again, by displaying an error message.

By way of example, even in a case where the puncture guideline generated by the image processor 42 has been first displayed at 67 degrees on the monitor 25 by the control circuit 6, and where the puncture needle has deviated from 67 degrees to 63 degrees, the image processor 42 executes the correction processing of the puncture guideline to 63 degrees by sensing the angle of the puncture needle, and the control circuit 6 redisplays the result on the monitor 25, so that the proceeding direction of the puncture needle can be displayed as the puncture guideline.

Besides, in a case where the puncture needle has greatly deviated as from 67 degrees to 50 degrees relative to the puncture guideline, the image processor 42 commands the monitor to display a message such as "Accurate puncture impossible" on the ultrasonic image. Accordingly, the operator having recognized the message obtains the chance of redoing the puncture.

Owing to such a display, the operator can confirm the conspicuous deviation of the puncture needle from the puncture guideline, and hence, any harm to the patient can be prevented from occurring.

FIG. 21 is a diagram showing a puncture guideline which is displayed on the monitor when a puncture needle has bent in the shape of a curve, in this embodiment.

As shown in FIG. 21, in the case where the puncture needle has bent curvilinearly, the curved puncture guideline is represented on the basis of image data (coordinate information on an ultrasonic image) which the control circuit 6 has obtained by performing a quadratic regression calculation or the like, as a correction method for the deviation of the puncture guideline.

Also on this occasion, the control circuit 6 executes the quadratic regression calculation on the basis of the positions (coordinates) of the puncture needle with the lapse of time, on the ultrasonic image as specified by the B-mode image signal. Besides, the control circuit 6 writes the coordinates on the ultrasonic image as obtained by the calculation, into the displaying image memory 23 as the curvilinear puncture guideline, and it causes the monitor 25 to display the information thereon. In this way, even when the puncture needle has bent curvilinearly, the control circuit 6 calculates the predictive course of the puncture needle, and the monitor 25 displays the course, so that the puncture guideline can be corrected by an inexpensive unit.

### (Seventh Embodiment)

Next, the seventh embodiment will be described. An ultrasonic diagnostic equipment according to this embodiment consists in that the puncture guideline emphasis display according to the sixth embodiment is performed in the imaging of the puncture mode or the manual puncture mode according to any of the first through fifth embodiments.

More specifically, the control circuit 6 controls the variable-gain amplifier 41, image processor 42, displaying image memory 23, image generation circuit 24, etc. so that the puncture guideline emphasis display according to the sixth embodiment may be executed using the image data which have been acquired in accordance with the imaging mode (automatic puncture mode or manual puncture mode) according to any of the first through fifth embodiments.

In a case, for example, where the puncture guideline emphasis display is performed in the imaging which employs the puncture mode according to the first embodiment, the processing shown in FIG. 17 is executed at the step S6 in FIG. 6. That is, the control circuit 6, image processor 42, etc. perform the peak holding process, the angle correction process for the puncture guideline, etc. by using the puncture mode image data and the manual puncture mode image data. Besides, the Doppler processing unit 50 detects the velocity component in the Doppler mode by using, at least, two reflection waves separate at least two rates (reflection waves corresponding respectively to the first and third transmission ultrasonic waves), for each scan line. The control circuit 6, image processor 42, etc. execute the recognition of the puncture needle, etc. by using the obtained velocity component.

According to such a configuration, while the motion artifacts ascribable to the motion of the puncture needle are appropriately imaged by the imaging mode according to any of the first through fifth embodiments, the accurate puncture guideline of high visibility can be displayed on the ultrasonic image by the emphasis display of the puncture guideline. As a result, this embodiment can contribute to further enhancement in the quality of medical service.
(1) The functions according to each of the embodiments can be incarnated in such a way that programs for executing the corresponding processes are installed in a computer such as workstation, and that the programs are expanded on memories. On this occasion, the programs which can cause the computer to execute the processes can also be distributed in a state where they are stored in a record medium such as magnetic disk (floppy disk, hard disk, or the like), optical disk (CD-ROM, DVD, or the like), or semiconductor memory.
(2) In each of the embodiments, the enhancement of the visibility of the puncture needle has been explained by way of example. However, it is not intended to restrict the invention to the puncture needle, but the invention can be exploited also in cases of observing the other clinically beneficial information items of the motion of a living body, etc.
(3) In each of the embodiments, the case where the three times of ultrasonic transmissions (or the three times of ultrasonic transmissions including the dummy rate) are performed for each of the plurality of ultrasonic scan lines has been explained by way of example. However, it is not intended to restrict the number of times of ultrasonic transmissions for each scan line, to three, but the invention may well be configured so as to execute a larger number of times of ultrasonic transmissions. Even in case of such a configuration, echo signals based on the two transmission ultrasonic waves which are temporally separate at least two rates are utilized, whereby the puncture needle, etc. can be imaged more appropriately than in the prior art.
(4) Needless to say, in all the embodiments, symmetry holds for the replacement of the polarities of the transmission ultrasonic waves (replacing all the positive polarities with negative polarities, or all the negative polarities with positive polarities), in view of the properties of the ultrasonic waves.

Besides, various inventions can be formed by appropriately combining a plurality of constituents disclosed in the embodiments. By way of example, some of all the constituents indicated in each embodiment may well be omitted. Further, the constituents over the different embodiments may well be appropriately combined.

### Industrial Applicability

According to the present invention described above, it is possible to realize an ultrasonic diagnostic equipment having a function which can enhance the visibility of, for example, a puncture needle in puncture under ultrasonic guide, and a method of controlling the ultrasonic diagnostic equipment.

## Claims

1. An ultrasonic diagnostic equipment wherein, in order to image an inner part of a patient, an ultrasonic transmission is executed at a first time interval into the patient, thereby to obtain a first image, comprising:
a transmission/reception unit which performs a plurality of ultrasonic transmissions into the patient at a second time interval longer than the first time interval, and which receives echo signals corresponding to ultrasonic waves of the respective transmissions, from the patient;
an image generation unit in which the echo signals corresponding to the respective transmission ultrasonic waves are added or subtracted therebetween, thereby to generate second image data; and
a display unit which displays a second image on the basis of the second image data.

2. An ultrasonic diagnostic equipment as defined in claim 1, wherein:
the first image is an image for imaging morphology of the patient; and
the second image is an image for imaging a puncture needle stuck into the patient.

3. An ultrasonic diagnostic equipment as defined in claim 1, wherein said display unit displays the first image and the second image simultaneously.

4. An ultrasonic diagnostic equipment as defined in claim 1, wherein the second time interval is integral times as long as the first time interval.

5. An ultrasonic diagnostic equipment as defined in claim 1, further comprising:
an input unit for inputting the second time interval;
said transmission/reception unit performing the plurality of ultrasonic transmissions into the patient at the second time interval inputted by said input unit.

6. An ultrasonic diagnostic equipment as defined in claim 1, wherein:
said image generation unit specifies a position of a tip of a puncture needle which is inserted into the patient, from at least one among brightness information of the second image, and Doppler information and position information which are based on a transmission ultrasonic wave being separate, at least, the second time interval; and
it generates the second image which contains a locus of the specified tip.

7. An ultrasonic diagnostic equipment as defined in claim 6, wherein said image generation unit performs a peak holding process for a signal corresponding to the tip, at the specified position of the tip, thereby to generate the second image which contains the locus of the specified tip.

8. An ultrasonic diagnostic equipment as defined in claim 7, wherein said image generation unit erases the locus of the tip based on the peak holding process, in a case where the second image which contains a movement displacement of the puncture needle coming near to a surface of the patient is generated using the Doppler information which is based on the transmission ultrasonic wave being separate, at least, the second time interval.

9. An ultrasonic diagnostic equipment as defined in claim 6, wherein said image generation unit generates the first image which contains a puncture guideline being a predictive traveling path of the puncture needle, on the basis of the locus of the tip of the puncture needle.

10. An ultrasonic diagnostic equipment as defined in claim 9, further comprising:
a report unit for reporting occurrence of a deviation to an operator in a case where the deviation between the puncture guideline and the locus of the tip of the puncture needle has exceeded a predetermined magnitude.

11. An ultrasonic diagnostic equipment comprising:
an ultrasonic probe which includes ultrasonic transducers that perform transmissions/receptions of ultrasonic waves in a plurality of ultrasonic scan directions within a patient in response to applied drive signals, respectively;
a transmission control unit which supplies the drive signals to the ultrasonic transducers so as to perform a plurality of times of ultrasonic transmissions which are taken with reference to a. predetermined rate period and which include at least one time of polarity inversion, in each of the plurality of ultrasonic scan directions;
a reception unit which receives a plurality of reflection waves based on the plurality of times of transmitted ultrasonic transmissions, with reference to the predetermined rate period;
an image generation unit in which an addition or subtraction process is performed using two of the plurality of reflection waves as are temporally separate, at least, 2 rates at the predetermined rate period, thereby to generate first image data; and
a display unit which displays a first image on the basis of the first image data.

12. An ultrasonic diagnostic equipment as defined in claim 11, wherein the first image is an image for imaging a puncture needle.

13. An ultrasonic diagnostic equipment as defined in claim 11, wherein:
said image generation unit performs an addition or subtraction process by using at least two reflection waves which are predetermined ones of the plurality of reflection waves, or which correspond to adjacent rates of the plurality of reflection waves, thereby to generate second image data; and
said display unit displays the first image and second image in parallel or in superposition, on the basis of the second image data.

14. An ultrasonic diagnostic equipment as defined in claim 11, wherein:
said transmission control unit executes the plurality of times of ultrasonic transmissions which include at least one dummy rate where no ultrasonic wave is transmitted;
said image generation unit performs the addition process by using two of the plurality of reflection waves as are temporally separate at least 2 rates at the predetermined rate period and as have opposite polarities, thereby to generate the first image data; and
it generates second image data on the basis of predetermined ones of the plurality of reflection waves; and
said display unit displays the first image and the second image in parallel or in superposition, on the basis of the second image data.

15. An ultrasonic diagnostic equipment as defined in claim 11, wherein said image generation unit generates the first image by using settings which are different from those of the second image as to a dynamic range, a gain, postprocessing, persistence and other image processing conditions.

16. An ultrasonic diagnostic equipment as defined in claim 13, wherein said display unit displays at least part of the first image in a color which is different from that of the second image.

17. An ultrasonic diagnostic equipment as defined in claim 11, wherein:
said transmission control unit executes the plurality of times of ultrasonic transmissions which include at least two times of polarity inversions; and
said image generation unit performs the subtraction process by using two of the plurality of reflection waves as are temporally separate at least 2 rates at the predetermined rate period and as have an identical polarity, thereby to generate the first image data.

18. An ultrasonic diagnostic equipment as defined in claim 11, wherein:
said transmission control unit executes the plurality of times of ultrasonic transmissions which include at least one dummy rate where no ultrasonic wave is transmitted; and
said image generation unit performs the subtraction process by using two of the plurality of reflection waves as are temporally separate at least 2 rates at the predetermined rate period and as have an identical polarity, thereby to generate the first image data.

19. An ultrasonic diagnostic equipment as defined in claim 11, wherein:
said transmission control unit executes the plurality of times of ultrasonic transmissions which include at least one dummy rate where no ultrasonic wave is transmitted; and
said image generation unit performs the addition process by using two of the plurality of reflection waves as are temporally separate at least 2 rates at the predetermined rate period and as have opposite polarities, thereby to generate the first image data.

20. An ultrasonic diagnostic equipment as defined in claim 11, wherein:
said image generation unit specifies a position of a tip of a puncture needle which is inserted into the patient, from at least one among brightness information of the first image, and Doppler information and position information which are based on two reflection waves being temporally separate at least 2 rates at the predetermined rate period; and
it generates the first image which contains a locus of the specified tip.

21. An ultrasonic diagnostic equipment as defined in claim 11, wherein said image generation unit performs a peak holding process for a signal corresponding to the tip, at the specified position of the tip, thereby to generate the first image which contains the locus of the specified tip.

22. An ultrasonic diagnostic equipment as defined in claim 21, wherein said image generation unit erases the locus of the tip based on the peak holding process, in a case where the first image which contains a movement displacement of the puncture needle coming near to a surface of the patient is generated using the Doppler information which is based on the two reflection waves being temporally separate at least 2 rates at the predetermined rate period.

23. An ultrasonic diagnostic equipment as defined in claim 20, wherein said image generation unit generates the first image which contains a puncture guideline being a predictive traveling path of the puncture needle, on the basis of the locus of the tip of the puncture needle.

24. An ultrasonic diagnostic equipment as defined in claim 23, further comprising:
a report unit for reporting occurrence of a deviation to an operator in a case where the deviation between the puncture guideline and the locus of the tip of the puncture needle has exceeded a predetermined magnitude.

25. A method of controlling an ultrasonic diagnostic equipment wherein, in order to image an inner part of a patient, an ultrasonic transmission is executed at a first time interval into the patient, thereby to obtain a first image, comprising:
performing a plurality of ultrasonic transmissions into the patient at a second time interval longer than the first time interval, and receiving echo signals corresponding to ultrasonic waves of the respective transmissions, from the patient;
adding or subtracting the echo signals corresponding to the respective transmission ultrasonic waves, between them, thereby to generate second image data; and
displaying a second image on the basis of the second image data.

26. An ultrasonic-diagnostic-equipment controlling method comprising:
supplying drive signals to ultrasonic transducers so as to perform a plurality of times of ultrasonic transmissions which are taken with reference to a predetermined rate period and which include at least one time of polarity inversion, in each of a plurality of ultrasonic scan directions within a patient;
receiving a plurality of reflection waves which are based on the plurality of times of transmitted ultrasonic transmissions, with reference to the predetermined rate period;
performing an addition or subtraction process by using those two of the plurality of reflection waves which are temporally separate at least 2 rates at the predetermined rate period, thereby to generate first image data; and
displaying a first image on the basis of the first image data.
